# EUROPEAN PATENT SPECIFICATION

(11) **EP 1 470 149 B1**
(45) Date of publication and mention of the grant of the patent: **11.11.2009**
(21) Application number: 03701377.8
(22) Date of filing: 27.01.2003
(51) Int. Cl.: C07J 9/00, C11B 13/00

(54) **DISTILLIATIVE PROCESS OF EXTRACTING AND PURIFYING PHYTOSTEROLS AND PHYTOSTANOLS FROM TALL OIL PITCH**
DESTILLATIVES VERFAHREN ZUR GEWINNUNG UND AUFTRENNUNG VON PHYTOSTEROLEN UND PHYTOSTANOLEN AUS TALLÖLPECH
PROCEDE D'EXTRACTION ET DE PURIFICATION DE PHYTOSTEROLS ET DE PHYTOSTANOLS EXTRAITS DE POIX DE TALL OIL

(30) Priority: 28.01.2002 US 60022
(43) Date of publication of application: 27.10.2004
(73) Proprietor: Cognis IP Management GmbH, 40589 Düsseldorf (DE)
(72) Inventor: SCHULTZ, Michael, E., Clear Lake Shores, TX 77565 (US); SONNIER, William, E., Pasadena, TX 77507 (US)
(74) Representative: Gittinger, Andreas
(86) International application number: PCT/CA2003/000098
(87) International publication number: WO 2003/064442

(56) References cited:
- WO-A-99/42471
- US-A- 2 835 682
- US-A- 3 556 827
- US-A- 3 649 580

## Description

### FIELD OF THE INVENTION

This invention relates to the separation and purification of unsaponifiables, such as phytosterols and phytostanols, from tall oil pitch.

### BACKGROUND OF THE INVENTION

Sterols are naturally occurring compounds that perform many critical cellular functions. Phytosterols such as campesterol, stigmasterol, campestanol and beta-sitosterol in plants, ergosterol in fungi and cholesterol in animals are each primary components of cellular and sub-cellular membranes in their respective cell types. The dietary source of phytosterols in humans comes form plant material i.e. vegetables and plant oils. The average Western diet contains about 60-80 mg of phytosterols per day, which can be contrasted with a vegetarian diet, which provides about 500 mg per day. Recently, these dietary plant sterols have received a great deal of attention because of their possible anti-cancer properties and their ability to decrease cholesterol levels when fed to a number of mammalian species, including humans.

It is generally accepted that phytosterols offer a unique combination of long-term safety, efficacy, and versatility in human treatment. The ongoing challenges with respect to phytosterols is in their isolation, extraction and purification from plant sources, and in determining additional sources which are cost-effective and manageable on a large-scale.

Traditionally, phytosterols have been isolated from sources such as corn oil, wheat germ oil, soya bean pitch and corn oil pitch. Similarly, tall oil pitch, which is obtained during the process of preparing paper from wood, particularly pine wood, has been used as a phytosterol source. Generally, in a process called the "Kraft Process", wood chips are digested or cooked for 2 hours at 170° C in aqueous liquor containing sodium hydroxide and sodium sulfide. The digestion delignifies the wood chips and gives rise to cellulose pulp, sodium rosin soaps, sodium fatty soaps, lignin degradation products and a number of other chemicals. The sodium rosin soaps, sodium fatty soaps and other hydrophobic compounds, which remain the in cooking liquor, are separated out by concentrating the liquor causing them to skim or float to the surface (hence the term "skimmings").

Skimmings generally comprise, along with sodium rosin soaps and sodium fatty soaps, hydrophobic compounds such as phytosterols, phytostanols, esters, fatty alcohols, waxes, and terpenes, collectively often referred to as the unsaponifiable fraction. After acidulation of the skimming, the result is crude tall oil. This is then distilled to remove the volatile materials leaving a "pitch" as the residue. Phytosterols and their saturated analogues can be isolated from either the skimming or the pitch. Oy Kaukas AB in Finland has been practising the commercial extraction of phytosterols from skimming soap since 1981. Exemplary patents in this area include: US Patent No. 4,044031 to Johansson et al. which describes a hexane extraction process for the removal of sterols from crude sulfate soap skimmings; US Patent No. 3,965,085 to Oy Kaukas which uses, for the isolation of phytosterols from soap, a mixture of solvents comprising hexane, acetone, methanol and water; and US Patent No. 5,770,749 to Kutney et al. which teaches a process of extracting sterols from pulping soap in which the solvent mixture comprises water, ketone, a hydrocarbon and no alcohol.

A number of researchers have attempted efficiently to extract phytosterols from pitch. In US Patent No. 2,715,638, Albrecht et al. teach the use of an amount of dilute alkaline solution to neutralize the fatty and rosin acids in the pitch but in an amount to saponify the sterol esters. The remaining organic phase is then separated and saponified with an alcoholic alkaline solution to covert the steryl esters into free sterols for subsequent dilution in hot water to precipitate the sterols by cooling.

United States Patent No. 3,840,570 to Jullan provides a process for preparing sterols from tall oil pitch by extraction in a water - alcohol - hydrocarbon mixture followed by saponification and subsequent purification. The starting material in this process is tall oil pitch from which are extracted phytosterols and various impurities. It is recognized that, in any tall oil pitch purification process, the long-chain alcohol and acid impurities are particularly difficult to separate from the sterols (which are, themselves, high molecular weight alcohols). This procedure is cumbersome as it involves several solvent extraction steps with different polar and non-polar solvents. Solvent recovery would be necessarily complex.

US Patent No. 6,297,353 to Diaz et al teaches a method of obtaining unsaponifiables from crude tall oil or its vacuum distillation products including, tall oil fatty acids, tall oil rosin acids, distilled tall oil or pitch which comprises:
1) "neutralizing" the starting material with sodium hydroxide and/or potassium hydroxide;
2) dehydrating/drying the mix to a level of humidity no more than 10%;
3) distilling in two short path distillation columns; and
4) collecting the soap free distillate and the neutral compound free residue.

In the Diaz examples, particularly 7 and 8, it appears that the residue from the distillation is extracted with hexane and ethanol, then titrated with sulfuric acid (column 11 lines 21-28 and column 12 lines 30-34). The authors claim not to saponify the starting material but rather to neutralize thereby maintaining as opposed to breaking the ester linkages.

Other researchers have addressed the issue of tall oil pitch processing: United States Patent No. 2,835,682 to Steiner and Fritz; United States Patent No. 2,573,891 to Christenson, US Patent No. 3,926,936 to Lehtinen, US Patent No. 4,524,024 to Hughes and US Patent No. 3,887,537 to Harada. In Harada, the pitch is first saponified with an alkali metal base and low molecular weight alcohol and then the mixture is introduced into a thin film evaporator to remove low boiling point matter such as water, alcohol and light unsaponifiables. The bottom fraction of the first evaporator is then fed into a second thin film evaporator in which the unsaponifiables, including phytosterols are removed as light ends and a molten soap is recovered as the bottom fraction. Lehtinen teaches the recovery of fatty acids and rosin acids by reacting the pitch with an alkali at 200-300° C, in the amount of 5 to 25% tall oil pitch, prior to vacuum distillation of the heated mixture to recover the fatty acids and rosin acids in the distillate fraction

It is an object of the present invention to obviate or mitigate the disadvantages and insufficiencies of the prior known processes.

### SUMMARY OF THE INVENTION

The present invention provides a process for isolating and purifying phytosterols and phytostanols from tall oil pitch which comprises:
a) feeding the pitch into a first distillation column;
b) distilling the pitch to remove excess rosin acids and fatty acids to form a distilled pitch;
c) saponifying the distilled pitch with an aqueous solution of one or more alkali metal bases to form a saponified pitch;
d) neutralizing the saponified pitch with an amount of acid sufficient to achieve an ending pH of between 5.8 and 6.3 thereby forming a neutralized pitch;
e) allowing the neutralized pitch to phase separate for a period of at least 12 hours or until the water content of the pitch, on phase separation, is less than 15%, thereby forming a settled, pitch and a water phase;
f) removing substantially all of the remaining water from the settled pitch to form a modified pitch;
g) distilling the modified pitch in a second distillation column to remove lights ends from the modified pitch and to produce a bottom fraction comprising free phytosterols and/or phytostanols;
h) distilling only the bottom fraction in a third distillation column to produce a light phase distillate comprising free phytosterols and/or phytostanols;
i) dissolving only the light phase distillate in a solvent comprising at least one alcohol to produce a solution of phytosterols and/or phytostanols;
j) cooling the solution to form a slurry with phytosterols and/or phytostanols crystallized therein; and
k) washing, filtering and drying the slurry to isolate the crystallized phytosterols and/or phytostanols from the filtrate.

The present invention also comprises compositions of phytosterols and/or phytostanols prepared according to the process described herein.

### BRIEF REFERENCE TO THE DRAWINGS:

Various aspects of the invention will be illustrated by the following non-limiting drawings wherein:
Figure 1 is a flow chart of an extraction and purification process from tall oil pitch in accordance with the present invention;
Figure 2 is a flow chart of an embodiment of the process of the present invention.

### PREFERRED EMBODIMENTS OF THE INVENTION

The present invention provides a unique method for the processing of tall oil pitch for the specific purpose of separating the phytosterols and phytostanols therefrom. The term "phytosterols and/or phytostanols" as used herein to refer to the mixture extracted and purified from tall oil pitch in accordance with the present invention, refers to a mixture, the majority of which comprises phytosterols and/or phytostanols. The term "tall oil pitch" or "pitch", hereinafter, is understood to mean the dark, tarry residue product of the distillation of crude tall oil, the latter having been produced by acidulation of skimming soap. Skimming soap, in turn, is produced by the evaporation of black liquor, one of the products of the "Kraft Process" in which wood chips are digested with an alkaline solution. The steps of producing pitch from wood chips are well known and practised in the field and will not be elaborated upon herein.

From a technical perspective, of all the wood or forestry derived products, it is the soap and the pitch that are most suitable for the isolation and purification of phytosterols. Both the soap and the pitch have their advantages and disadvantages as starting materials. During high temperature distillation of tall oil, some thermal decomposition of the volatile constituents or phytosterols may occur, with the result that these decomposed substances remain in the pitch. Nonetheless, two advantages of the pitch which cannot be overlooked are that it has a higher sterol concentration than the soap (up to 18% of its mass) and it has an up to six times smaller volume in comparison with the soap which makes it more economical to work with. Within the scope of the present invention, there is provided an extraction and purification process which is effective to produce good yields of phytosterols from the pitch at the high purities required for pharmaceutical, nutraceutical and food uses.

The wood chips from which the starting material (pitch) of the present invention originally derived may be from any hard wood or soft wood variety of tree including, but not limited to, fir, cedar, pine, spruce, oak, hemlock and poplar. Most preferably, the chips from which the pitch is produced are from any Pacific Northwest or Southeast American or European forest variety of woods.

Tall oil pitch is known to include a variety of free and esterified phytosterols including, but not limited to sitosterol, stigmasterol, campesterol and their saturated equivalents. Free sterols and stanols, once extracted and purified from pitch can be used as is in pharmaceuticals, nutraceuticals, foods, beverages and the like or can be chemically modified to confer properties such as stability or solubility. Examples of such manipulation are provided in WO-A-01/00653, WO-A-00/78789, WO-A-01/32679, WO-A-99/63841, WO-A-01/66560, and US Patent No. 6,087,353, all of which are assigned to Forbes Medi-Tech Inc.

It is known to extract sterols from pitch using an initial saponification step followed by a two-stage distillation: US Patent No 3,887,537 to Harada. In addition, it is known to extract sterols from pitch by a process which involves the following steps:
1) an alkali base metal is added to the pitch and mixed at elevated temperatures to saponify;
2) a strong mineral acid is then added to the saponified pitch to neutralize;
3) the neutralized pitch is then heated under vacuum to remove excess water--net result is a "modified pitch";
4) this modified pitch is added to a low pressure wiped film evaporator to remove the "light ends" of the pitch. The bottom portion comprises the phytosterols.
5) the bottom portion is moved to a second, this time "thin film" evaporator to distill the phytosterols into a "light phase" distillate;
6) the light phase distillate is then heated and stirred in alcohol to dissolve the phytosterols.
7) the solution is cooled and mixed at high speed producing a slurry which is cooled, washed and filtered to dry. Phytosterol crystals are recovered.

The family of applications covering this process includes Canadian Patent Application No. 2,230,373 and WO-A-99/42471, all of which are owned by the present assignee. What is provided within the scope of the present invention, however, is a superior process which surpasses all other known pitch extraction and purification processes in terms of sterol yield and efficiency. The changes that distinguish the present invention from the disclosure and claims in WO-A-99/42471 are not merely routine but represent significant improvements, heretofore unrecognized, as will become apparent below.

The first step of the process of the present invention, which is critical, is feeding the pitch into a first distillation column and thereafter distilling the pitch. This preliminary distillation step serves two purposes. Firstly, excess rosin acids and fatty acids are removed thereby making the subsequent saponification step more efficient in terms of conversion of steryl esters to free sterols. Secondly, this distillation reduces the amount of alkali metal base required for saponification and the amount of acid required for the subsequent neutralization. In a preferred embodiment, the pitch is distilled to achieve an acid value of less than 40, most preferably less than 30. This step is particularly important in pitch sources having high acid values. The distillation column may be selected from the group consisting of short path distillation columns, wiped film evaporation columns, thin film evaporation columns and molecular distillation columns. In a preferred embodiment, the distillation column used in this step is a wiped film evaporation column. No where in any of the prior processes is this pre-treatment of the pitch used.

The second step of the process of the present invention is saponification of the distilled pitch by adding, an aqueous solution of one or more alkali metal bases. Preferred bases include sodium hydroxide, potassium hydroxide or combinations of both. Although to some measure dependent on the pitch source, the weight percentage of alkali metal to pitch should be in the range of 1% to 30%. More preferably, the range is 1 to 10% or 1 to 15% on an anhydrous basis. For pitch sources with high acid values, a more alkali metal base and longer reaction times must be used in order to attain satisfactory conversion of the steryl ester to free sterol. For example, a pitch source with an acid value of 50 would require that the weight percentage of alkali metal to pitch be in the range of 20% to 25%. Preferably, saponification is conducted at a temperature in the range of 100°C to 250°C for a period in the range of 60 to 300 minutes, more preferably 120-240 minutes.

The third step is neutralizing the saponified pitch with one or more acids to achieve an ending pH of between 5.8 and 6.3. Achieving a pH within this range is critical. A higher pH will result in difficulty in the subsequent water removal steps. A lower pH will catalyze the reversion of the free sterols to their esterified form during storage and handling thereby significantly reducing end yield of free sterols. Preferably, the neutralization is carried out at a temperature in the range of 90°C to 130°C, more preferably in the range of 100°C to 120°C, more preferably 101°C to 120°C and most preferably 105°C to 118°C for a period of 1 to 10 hours. Temperatures in the higher end of the range are preferred as this reduces viscosity and allows for more efficient mixing of the pitch and acid thereby facilitating more rapid neutralization. Acids which may be used include all organic and mineral acids, including, but not limited to sulfuric acid, hydrochloric acid, phosphoric acid or any combination thereof. In a preferred embodiment, the neutralization step is carried out under vigorous agitation and/or mixing. Both batch and continuous processes may be used. In the case of a continuous system, it is preferred to use a mixer at this stage. No disclosure of the preferred higher neutralization temperature is made in WO-A-99/42471.

The fourth step is allowing the neutralized pitch to settle for a period of at least 12 hours or until the water content of the pitch, on phase separation, is less than 15%, thereby forming a settled pitch and a water phase. In this step, water added during the prior steps of saponification and neutralization is removed, preferably by phase separation and evaporation (stripping). This settling can be achieved by holding the neutralized pitch in a vessel for the required period of time without agitation. Settling may occur in the same vessel as the prior saponification and neutralization steps or in a separate vessel. What is critical is that the water content on completion of this step must be less than 15%, more preferably less than 10%, otherwise the pitch will be too viscous after final water removal and to difficult to process further downstream due to the high neutralization salts content. It is preferred that the temperature for this settling step is maintained at the temperature of the prior neutralizing step. This type of settling step or an appreciation of its' benefits are not described in any of the prior publications.

Additionally, there may be included a second phase separation step, wherein the water phase removed in the first settling process is transferred to another vessel for secondary phase separation. During the first phase separation step, frequently a "rag" layer or emulsion exists between the water phase and the organic phase, which should be removed to improve the quality of the pitch before the downstream distillation steps. In the secondary vessel, the rag layer is allowed to separate by further "settling" without agitation. It is preferred that the temperature for this second phase separation step is maintained at the temperature of the prior settling step

Following the settling step(s), and notwithstanding the removal of water in the prior step, the pitch must be further dehydrated i.e. substantially all of the remaining water from the settled pitch must be removed. This may be achieved by any means which facilitates bulk disengagement of water from the organic phase. For example, heating at a sufficient temperature or heating under vacuum conditions. Most preferably, water may be removed using a pressure strip wherein the pressure is no greater than atmospheric and the temperature is maintained below 105°C in order to prevent reversion of the free sterols to steryl esters as the water is removed.

It is preferred that the temperature of the pitch after this dehydration step be cooled to less than about 80°C, as above noted, to avoid reversion of the free sterols to steryl esters. Furthermore, if the dehydrated pitch is to be stored prior to the initiation of the downstream processing steps, it should be cooled and maintained at a temperature of approximately 60°C or less.

All of the steps, heretofore described, relate to various pre-treatments of the "raw" pitch (residue of crude tall oil distillation) before it is subject to the subsequent extraction steps. The particular way in which the pitch is modified is critical to the success of the process of the present invention. Accordingly, the term "modified" pitch, with the scope of the present invention, refers to a pitch which has, at the very least, been subject to:
- pre-saponifcation distillation, preferably to achieve an acid value of less than 40
- saponification, preferably at a temperature in the range of 100°C to 250°C for a period in the range of 60 to 300 minutes
- neutralization to achieve a pH in the range of 5.8 to 6.3, preferably at a temperature exceeding 100° C and with vigorous agitation
- at least one settling/phase separation, and
- dehydration preferably at a pressure no greater than atmospheric pressure and at a temperature below 105°C

Following dehydration, the "modified" pitch is in a form most appropriate and effective for the two subsequent vacuum distillation steps (the second and third of the process) which follow. Falling short path distillation columns with or without scraper, flat, rotary or others, short path distillation column centrifuges, multi-stage short path distillation columns, molecular distillation columns, wiped film evaporation columns, and thin film evaporation columns are all adequate for use within the present invention. In a preferred mode, wiped film evaporators or short path distillation columns are used. Distillation conditions are described in more detail below. In one embodiment, a degasser may be operated just prior to the second stage of evaporative distillation to assist in the removal of residual water and light boiling point components.

The light phase distillate resulting from the third distillation comprises free phytosterols and/or phytostanols. Subsequently, it is immediately dissolved in a solvent comprising at least one alcohol to produce a solution of phytosterols and/or phytostanols. The solution is then cooled to form a slurry with phytosterols and/or phytostanols crystallized therein and lastly, the slurry is washed and filtered to isolate the crystallized phytosterols and/or phytostanols from the filtrate. Preferably, the solvent used to dissolve the light phase distillate comprises at least one low molecular weight monohydric alcohol. Suitable solvents include, but are not limited to, alcohols such as methanol, ethanol, isopropanol, and acetate esters thereof, ketones such as acetone, methyl ethyl ketone (MEK), methylisobutylketone (MIBK), C1 to C8 hydrocarbons or mixtures thereof.

### Flow Diagrams:

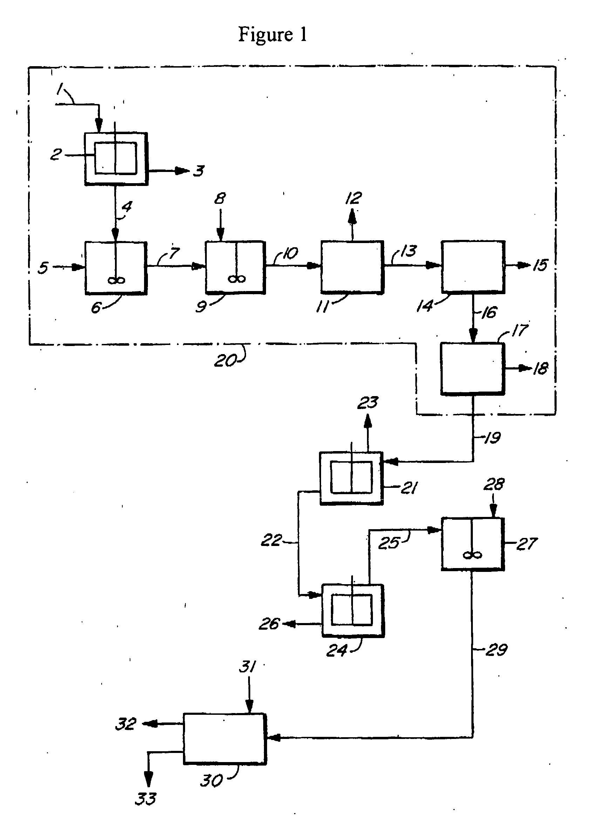

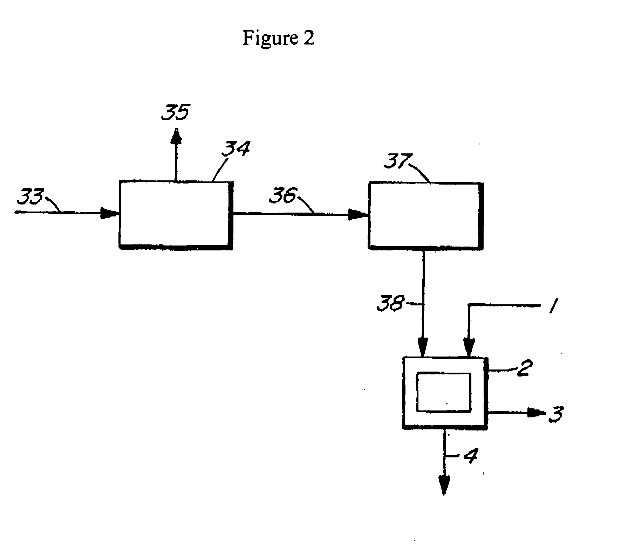

In operation, and with reference to Figures 1 and 2, wherein like numerals throughout refer to the same element, the preferred process of the present invention is as follows:

Within block 20, there is provided the series of steps required to produce a "modified pitch" 19 in accordance with the present invention. Tall oil pitch 1 is introduced into a wiped film evaporator 2 and distilled to remove excess fatty acids and rosin acids 3, thereby producing distilled pitch 4. Preferably, this evaporator operates in the range of 100 to 10,000 microns pressure and at a temperature in the range of 240° to 285°C. Generally, depending on the flow rate and the pitch source, it takes less than a minute to achieve the desired acid value of 40 or less. The acid value of the pitch may be assessed by taking a sample and titrating.

Distilled pitch 4 is then added with an alkali metal base 5 into reactor 6. The amount of alkali metal base relative to the distilled pitch preferably should be sufficient to facilitate substantially complete or complete saponification of the distilled pitch. Generally, a water solution of an alkali metal base such as sodium hydroxide, potassium hydroxide or a combination whereof is preferred. These compounds or combinations will provide a relatively high alkalinity for a relatively reasonable cost. If such compounds or combinations are used, then the stoichiometric proportion of alkali metal base 5 to distilled pitch 4 theoretically that is required to achieve complete conversion typically may be approximately 1% by weight. Factors that may impact the precise amount of base to be used include the specific characteristics of tall oil pitch 1 and distilled pitch 4 (which characteristics may differ from batch to batch and source to source) and most importantly, the acid value of the tall oil pitch. For pitch sources which have a high acid value, more base and a longer reaction time are required in order to attain satisfactory conversion of the steryl esters to free sterols.

What is important to note, however, is that the amount of base required at this saponification step, within the scope of the present invention, is less than that required in prior saponification processes due to the fact that tall oil pitch 1 is distilled before saponification. This is due, in part, to the fact that, in prior known processes, a significant amount of the base is consumed in reaction with other components of the pitch such as rosin acids and fatty acids 3. A substantial portion of these unwanted components are removed in evaporator 2 freeing the base to react with the steryl esters. Nonetheless, to provide a strong driving force for the saponification reaction and to ensure substantially complete or complete conversion of the steryl esters to free sterols, the proportion of alkali metal base 5 to distilled pitch 4 may be in the range of 1 to 15% by weight. In a preferred embodiment, distilled pitch 4 is saponified with 50% caustic (sodium hydroxide) diluted to a concentration of from between 7 to 12%, most preferably 9.5%.

Mixing is sustained in a reactor 6 with sufficient vigor to maintain contact between distilled pitch 4 and alkali base metal 5. Typically, an operating temperature in the range of 100° to 250°C, more specifically from 120° to 160°C and most specifically 145°C for a period of time in the range of 120 to 240 minutes will suffice to facilitate the desired conversion.

Following saponification in reactor 6, saponified pitch 7 is discharged into second reactor 9 for the neutralization step. Acid 8 is added to reactor 9 for this purpose. In an alternative embodiment, this neutralization step occurs in reactor 6 (acid 8 is added directly to reactor 6), the same vessel as used in the saponification step. Either way, it is critical that the ending pH of this step fall within the range of 5.8 to 6.3. A higher pH will result in difficulty in the subsequent water removal steps. A lower pH will catalyze the reversion of the free sterols to their esterified form during storage and handling thereby significantly reducing end yield of free sterols. Preferably, the neutralization is carried out at a temperature in the range of 90°C to 130°C, more preferably in the range of 100°C to 120°C. for a period of 1 to 10 hours. It is most preferred that the temperature be greater than 100°C. Temperatures in the higher end of the range are preferred as this reduces viscosity and allows for more efficient mixing of the pitch and acid thereby facilitating more rapid neutralization. Continuous as opposed to batch neutralization is preferred.

Acid 8 may be a simple organic acid such as acetic acid or formic acid, both of which are commercially practical. Alternatively, acid 8 may be a mineral acid such as sulphuric acid, hydrochloric acid or phosphoric acid. These are relatively strong mineral acids and are favoured over weaker acids such as boric acid. In a preferred embodiment, acid 8 is either 75% or 85% phosphoric acid or 93-98% sulphuric acid.

Accordingly, sufficient acid is added to reactor 9 (or reactor 6 if one vessel used for both steps) to achieve a water phase pH between 5.8 and 6.3 thereby yielding neutralized pitch 10. Monitoring is required in order to achieve this pH. It is most preferred that this neutralization step is carried out under vigorous agitation, at a temperature in the range of 90°C to 105°C for a sufficient period of time to achieve a pH within the desired range. Timing varies considerably depending on whether the process is batch or continuous. For example, the former may take only minutes to achieve the desired pH, the latter, one or more hours.

Neutralized pitch 10 is introduced into settling vessel 11 and held with no agitation for a period of at least 12 hours or until the water content of the neutralized pitch is less than 15%, preferably less than 10% by weight thereby yielding settled pitch 13 and water phase 12, the latter of which is discarded. Water content may be measured by Krlki titration. Alternatively, this settling step may occur in either reactors 6 or 9. The key factors are that there is no agitation in the vessel and that the water content of the pitch be sufficiently reduced, to at the least within the parameters described herein. In a most preferred embodiment, settled pitch 13 is subject to a secondary phase separation in receiving tank 14 to remove rag layer 15 and yield ultra-settled pitch 16. Once again, the pitch is held without agitation and for a period of 2 to 24 hours depending on the vessel size and geometry.

Notwithstanding the significant and critical removal of water during the one or two prior phase separation steps, some water remains in the ultra-settled pitch which must be removed or substantially removed before the downstream distillation steps. Accordingly, ultra-settled pitch 16 is transferred to reactor 17 and is subject to either vacuum or atmospheric pressure stripping i.e. to strip or evaporate remaining water thereby yielding modified pitch 19. In one embodiment, the temperature in reactor 17 is maintained below 105°C and water 18 is removed by atmospheric stripping. This may be batch or continous, although the latter is preferred. Temperatures below 105°C are preferred for this embodiment, as higher temperatures will cause reversion of some sterols to their ester form. In another, although less preferred, embodiment, water 18 is removed by vacuum stripping ultra-settled pitch 16 i.e. heating the pitch (up to approximately 149°C) until the water content is less than 1%.
owing this water strip, modified pitch 19 is immediately cooled to a temperature of less than about 80°C in order to prevent or minimize reversion of free sterols to steryl esters.

Modified pitch 19 is introduced into a low pressure wiped film evaporator 21 for the removal of light ends 23. These light ends will comprise the fatty acids and rosin acids which were not removed in the pre-saponification distillation. The bottom fraction 22 contains the free phytosterols and is removed from evaporator 21 and moved into a second low pressure wiped film evaporator 24. Evaporator 24 serves to distill free phytosterols present in fraction 22 into light phase distillate 25. Distillate 25 also comprises fatty alcohols, fatty acids, rosin acids and high molecular weight wax esters. A bottom fraction 26 is stored and may be used as a fuel or feedstock for other industries.

The specific reaction conditions (for example: temperature, pressure and time of residency) within each of evaporators 21 and 24 will vary depending on the characteristics and type of the source pitch as well as on the type of evaporator employed. These conditions may also vary depending on whether the modified pitch is "degassed" prior to the introduction of modified pitch 19 into evaporator 21. In a preferred embodiment, a degasser is used (not shown in figures) to remove residual water and other light boiling point components. Generally, the degasser operates at approximately 1000 to 10,000 microns and 100°C to 175°C. The preferred range of distillation conditions within evaporator 21 are as follows: temperature from 190°C to 230°C and pressure from 1000 to 15,000 microns. The preferred range of distillation conditions within evaporator 24 are as follows: temperature from 250° to 315°C and pressure from 100 to 5,000 microns

During this two-stage distillation, there are factors which may significantly increase end product purity. Firstly, control of the feed rate into evaporators 21 and 24 is important due to the potential fouling of the cold traps within the evaporators that protect the vacuum systems. Too high a feed rate will foul the cold traps and cause operation interruptions. Too low a feed rate will make the process commercially non-viable. However, it is well within the purview of one skilled in the art to maximize these conditions.

Secondly, control of the sterol content in the distillation cuts produced in evaporators 21 and 24 may be measured by GC analysis and this information used to control temperature and feed rate. Once again, it is well within the purview of one skilled in the art to maximize these conditions.

Light phase distillate 25 is introduced into a further reactor 27 where it is heated and stirred until dissolution has occurred in an added solvent 28. Solvent 28, as noted above, may include alcohol and may include water. Effective dissolution of free sterols, has been found to occur at over 65°C, more preferably over 70°C and using a solvent to distillate ratio of 0.5-1.5. Other temperatures may be used, however the solubility of phytosterols will decrease as the temperature is lowered. To some extent, the preferred temperature depends on the solvent to distillate ratio. The more solvent that is used, the lower the distillation temperature and vice versa. However, a higher solvent amount negatively impacts yield so a balance must be sought. It is well within the purview of one skilled in the art to find this "balance".

When dissolution has occurred, yielding a solution of phytosterols and phytostanols, the latter is cooled in reactor 27, thereby forming a slurry with the phytosterols and phytostanols crystallized therein. Typically, the temperature at which crystallization is effected may be in the range of 0 to 40°C (the more impurities, the higher temperature required for crystallization).

The cooled slurry 29 is washed and passed through a commercial filtration apparatus 30 advantageously using added solvent 31. In a preferred form, solvent 31 is the same solvent as used in the crystallization step (solvent 28), the solvent wash is at ambient temperature and the wash ratio is 0.5:1 to 1.5-1 relative to the slurry flow rate to apparatus 30. Drying occurs on a continuous dryer yielding purified phytosterols and phytostanols 32 and spent filtrate 33.

In a further aspect of the present invention, and as depicted in Figure 2, spent filtrate 33 is recovered, stripped of solvent 35 in reactor 34 (a solvent recycle system) yielding stripped filtrate 36, and then thermally treated to convert any free sterols to steryl esters in vessel 37. The product 38 of this recycling process can then be fed concurrently into pre-saponification distillation column 2.

It is to be understood that the entire process steps described herein may be effected in batch or continuous format.

### Example 1 Preparation of "Modified Pitch"

861 g Tall Oil Pitch (TOP, B.C. Chemicals, Northwest Canada origin) containing 17.1% total sterols (as fatty- and rosin-acid esters), 172 g NaOH (50% solution) and 640 g deionized water were charged to a 2-liter laboratory autoclave reactor (Autoclave Engineers) equipped with a mechanical agitator and electric heating mantel. The contents were heated to 140 - 160°C and held for 1 hr. with stirring. Resultant pressure of 40 - 60 psig was observed. The mass was cooled to 95°C and 124 g phosphoric acid (85%) was added. A slight exotherm related to the neutralization was observed and the mass was further heated to 110°C to facilitate mixing. The mass was stirred 30 min. after which it was allowed to settle, unstirred, for 2 hr. while maintaining temperature at 100 - 110°C. The temperature was reduced to 95°C and the aqueous heavy phase removed via bottom valve. 754 g of clear, yellow to brown aqueous brine containing primarily neutralization salts was removed until the phase boundary of the tarry organic light phase was just observed. 1014 g of organic light phase containing the hydrolyzed sterols, fatty- and rosin-acids, residual water and salts of neutralization was discharged to a 2-liter, 3-neck, glass round-bottom flask equipped with condenser, agitator and electric heating mantel. The neutralized mass was heated, with stirring, to 105°C where residual water began to boil,away. Temperature was gradually increased to 135°C during which approximately 100 ml water was collected. A vacuum was gradually pulled on the system by means of a mechanical pump. An end vacuum of 27 in. Hg. was attained during which an additional 25 ml of water was collected. The vacuum was relieved and products collected. A total of 131 g stripped water and 879 g modified pitch were obtained. GC analysis of the modified pitch showed 154 mg/g (15.4%) sterols as the free alcohol. The modified pitch thus obtained is suitable as a feed for high vacuum distillation equipment for the purpose of further enriching the sterol component.

### Example 2 Distillation of Modified Pitch

The modified pitch produced by the method of Example 1 was charged to the feed pot of a laboratory-scale short-path distillation apparatus (UIC model KD-6, stainless steel). This apparatus is well know to those practiced in the art and consists of a heated cylindrical evaporator and coaxial condenser. A mechanical vacuum pump provides the means to operate the system at reduced pressures in the range 0.01 mbar to 1,000 mbar. A wiper mechanism spreads the liquid feed material into a thin film on the evaporator surface thereby effecting a portion of the feed material to be evaporated. The vaporized feed material is conveyed by gaseous diffusion to the coaxial condenser surface where it is condensed to a liquid phase known heretofore as distillate. The non-vaporized portion of the feed, known heretofore as residue, flows by gravity and the pumping action of the wipers to a collection point where it is removed from the system by means of a mechanical pump. Similarly, the distillate is collected and removed. The thus described apparatus therefore provides a means of separating a multi-component feed into distillate and residue fractions based upon the various boiling points and gas-liquid equilibrium behavior of the components. Additional evaporation steps may be employed to separate the distillate and/or residue into further fractions. These additional steps may achieved by employing additional short-path distillation apparatus or by passing the desired fractions through a single short-path unit in batch-wise fashion.

Approximately 1 gal. (ca. 3,500 g) of modified pitch was fed continuously to the heated evaporator which was operated in the range 160 - 180°C and at a pressure in the range 3 - 5 mbar. The feed rate was approx. 3,500 g/hr. A distillate to residue ratio of 5:95 was achieved. This initial separation of the feed into distillate fractions of approximately 2 - 10% and corresponding residue fractions of 90 - 98% is advantageous in that is removes low levels of residual water not removed in previous drying steps and also separates certain low-boiling components of the pitch which are deleterious to subsequent distillation and purification steps. The optimal distillate to residue ratio of this initial separation, known heretofore as the pre-cut, is variable and depends upon the levels of these light-boilers in the feed. The relatively mild conditions and low mass fraction of distillate taken in the pre-cut results in only minimal sterol losses.

The 95% residue fraction obtained in the pre-cut operation was again fed to the short-path distillation apparatus. Evaporator temperatures in the range 240 - 260°C and pressures in the range 0.5 - 1.5 mbar were employed to achieve a distillate to residue ratio of approximately 40:60. This nominal 40% distillate fraction is variable and must be determined experimentally for various tall oil pitch feedstocks. In the present example the nominal 40% distillate fraction was analyzed by GC to contain 28.2% free sterols. The thus obtained distillate is suitable for subsequent purification by solvent crystallization. It is to be understood that higher recoveries of sterol in the distillate fractions are possible, but higher levels of co-boiling impurities which may also be present in the distillate, may have a deleterious effect on the purity of the final product obtained.

### Example 3 Crystallization of Sterol-Enriched Distillate

In a 250 ml Erlenmeyer Flask, 40.0 g of the the nominal 40% distillate obtained in Example 2 were combined with 60.0 g of a solvent mixture comprised of 80%methanol and 20% iso-propanol. The mixture was heated on a hot plate to just below boiling (ca. 60 - 65°C) thereby effecting complete dissolution of the distillate into the solvent resulting in a clear caromel-colored liquid with no visible solids. The solution was allowed to cool to room temperature (ca. 20 - 25°C) with occasional swirling. Needle-like crystals began to form in the solution at approximately 55°C which rapidly attained a thickened slurry-like character. The room temperature slurry was vacuum filtered on a Buchner Funnel and the resultant filter cake washed with two 30 ml portions of the solvent blend. The shiny, white crystals were dried in a vacuum oven at 70°C for 1 hr. to yield 8.3 g of dry product. GC analysis showed 96.3% sterols. Melt point was 138.3°C.

### Example 4 Industrial-Scale Preparation of Modified Pitch

In a process analogous to Example 1, 13,304 lb. of Tall Oil Pitch (TOP, B.C. Chemicals, Northwest Canada origin), 2460 lb. liquid caustic (50% industrial grade) and 10,350 lb. of water (municipal potable) were charged to a 4,000 gal. stainless steel reactor. The reactor was heated by means of steam pressure on an internal heating coil to a final temperature of 292°F over a period of one hour. This temperature was maintained for a period of 3 hr. Moderate agitation was maintained to ensure all phases remained well-dispersed. The temperature was decreased to approx. 200 - 210°F over a one hour period by means of cooling water on the internal coil. 2589 lb. of 75% phosphoric acid was charged. Mixing was maintained for one hour after acid addition. After 30 min. settling time, an aqueous sample was taken from the bottom outlet of the reactor. The pH of the sample was 5.9. The neutralized saponification batch was transferred forward to a 16,000 gal. settling tank. A second identical saponified and neutralized batch was prepared and transferred to the settling tank. The combined batches were allowed to settle for 12 hr. Temperature was maintained at 190 - 200°F during the settling period. At the conclusion of the settling period, an aqueous brine heavy phase was drained from the tank while an operator monitored a sight glass to detect the phase interface. A total of 4985 gal. aqueous brine layer was removed. The remaining organic layer was sampled for water content and showed 4.2% water by Karl Fischer Titration. The organic layer was heated at atmospheric pressure by means of steam pressure on an internal coil to a final temperature of 235°F. The residual water was flashed off and condensed by means on an overhead condenser. Stripping time was approx. 2 hr. A final sample of Modified Pitch was taken and showed a water content of 0.98%. Further gas chromatigraphic analysis showed 16.84% total sterols (esters and alcohol combined) and 15.16% free sterols (alcohol form only).

### Example 5 Industrial-Scale Distillation of Modified Pitch

Modified Pitch such as that prepared in Example 4. was fed continuously to a two-stage short-path distillation system (UIC KD-1200, 12 m² evaporator surfaces). The system is connected serially, with the residue (heavy) stream from the first stage feeding forward to the second stage. The temperature of the evaporating surfaces is controlled by means of a hot-oil circulation system. The condensing surfaces for each stage may be controlled independently by means of a tempered-water circulation system. Vacuum may be drawn on either stage independently by means of mechanical vacuum pumps equipped with 2-stage Roots-type boosters. Residual water and dissolve gasses are disengaged from the feed prior to the first evaporator stage,by means of a flash tank. Prior to the flashing, the temperature of the feed may be heated above its storage temperature by means of a steam-heated shell and tube heat exchanger. The following process parameters represent average values over a 24 hr. continuous period:

| | | |
|---|---|---|
| Feed Temp: | 278°F | |
| Feed Rate: | 33.5 lb/min. | |
| Evap. 1 Temp: | | 380°F |
| Evap. 1 Pressure: | | 4.5 mm Hg abs. |
| Cond. 1 Temp: | | 75°F |
| Dist. 1 Flow: | 3.4 lb/min | |
| Residue 1 Flow: | | 30.1 lb/min. |
| Evap. 2 Temp: | | 511°F |
| Evap. 2 Pressure: | | 1.6 mm Hg abs. |
| Cond. 2 Temp: | | 113°F |
| Dist. 2 Flow | 13.6 Ib/min. | |
| Residue 2 Flow: | | 16.5 Ib/min. |

The thus-obtained distillate of the No. 2 evaporator effect was subsequently analyzed by GC and found to contain 22.6% free sterol.

### Example 6 Industrial-Scale Crystallization of Sterol-Enriched Distillate

20,000 lb. of sterol-enriched distillate such as produced in Example 5 was charged to a 6,500 gal. jacketed stainless steel stirred-tank reactor. 20,000 lb. of a solvent blend of composition 80% methanol and 20% iso-propanol was likewise charged. The mixture was heated by means of steam pressure on the jacket to a temperature of 160°F and held for 1 hr. under moderate agitation. The heated mixture was fed at 100 lb/min to a two-stage continuous scraped-surface crystallizer (Armstrong) of stainless steel construction. The first crystallizer stage was cooled by means of cooling-tower water of 78°F average temperature on its jacket. The second stage jacket was connected to a chilled-water system maintained at 45°F. Average interstage temperature of the crystallizer mass was 122°F. Average exit temperature was 85°F. The thus-crystallized slurry was collected in a 6,500gal. stainless steel buffer tank equipped with agitator and recirculation pump. The slurry was fed to a continuous, vapor-tight vacuum-belt filter (Pannevis) at an average rate of 35 lb/min. The vacuum action of the filter serves to disengage the sterol crystals from the mother liquor. Various devices such as overflow weirs and spray bars enable the filter cake to be washed with fresh solvent. The filter cake is washed and dried to an average moisture of 30-40% before being discharged from the filter via a rotary airlock valve. The discharged wet cake is fed directly to a continuous rotary tray dryer (Wyssmont Turbo-Dryer) whereby the residual solvent is removed to a level of < 1%. The dried sterol crystals are discharged from the dryer via a second rotary airlock valve where they may be collected in a suitable container or further processed. In the present case, 3088 lbs. of dried sterol crystalline powder was obtained. GC analysis showed 96.2% sterols. Melt point of the sterols was 138.4°C.

## Claims

1. A process for isolating and purifying phytosterols and phytostanols from tall oil pitch which comprises:
a) feeding the pitch into a first distillation column;
b) distilling the pitch to remove excess rosin acids and fatty acids to form a distilled pitch;
c) saponifying the distilled pitch with an aqueous solution of one or more alkali metal bases to form a saponified pitch;
d) neutralizing the saponified pitch with an amount of acid sufficient to achieve an ending pH of between 5.8 and 6.3 thereby forming a neutralized pitch;
e) allowing the neutralized pitch to phase separate for a period of at least 12 hours or until the water content of the pitch, on phase separation, is less than 15%, thereby forming a settled pitch and a water phase;
f) removing substantially all of the remaining water from the settled pitch to form a modified pitch;
g) distilling the modified pitch in a second distillation column to remove lights ends from the modified pitch and to produce a bottom fraction comprising free phytosterols and/or phytostanols;
h) distilling only the bottom fraction in a third distillation column to produce a light phase distillate comprising free phytosterols and/or phytostanols;
i) dissolving only the light phase distillate in a solvent comprising at least one alcohol to produce a solution of phytosterols and/or phytostanols;
j) cooling the solution to form a slurry with phytosterols and/or phytostanols crystallized therein; and
k) washing, filtering and drying the slurry to isolate the crystallized phytosterols and/or phytostanols from the filtrate.

2. The process of claim 1 wherein, in step b), the pitch is distilled to achieve an acid value of less than 40.

3. The process of claim 1 wherein, in step b), the pitch is distilled to achieve an acid value of less than 30.

4. The process of claim 1 wherein the distillation columns in steps b), g) and h) are selected from the group consisting of short path distillation columns, wiped film evaporation columns, thin film evaporation columns and molecular distillation columns.

5. The process of claim 1 wherein the distillation column in steps b), g) and h) is a wiped film evaporation column.

6. The process of claim 1 wherein there is provided an additional, concurrent feed into the first distillation column, said feed being the filtrate from step k), **characterized in that** the filtrate is pre-treated to strip it of solvents and to convert substantially all the free sterols therein to steryl esters.

7. The process of claim 1 wherein, in step d), the saponified pitch is neutralized at a temperature exceeding 100°C for a period of from 1 to 10 hours.

8. The process of claim 1 wherein steps c) and d) occur in the same reaction vessel.

9. The process of claim 1 wherein step d) is carried out under vigorous agitation.

10. The process of claim 1 wherein in step d) the acid and pitch are mixed.

11. The process of claim 1 wherein step e) is carried out without agitation.

12. The process of claim 1 wherein there is provided an additional step, after step e) which comprises subjecting the water phase to a second phase separation.

13. The process of claim 1, **characterized in that** the step of removing substantially all of the remaining water from the settled pitch in step f) to form a modified pitch comprises the use of a water strip wherein the pressure is no greater than atmospheric and the temperature is below 105°C.

14. The process of claim 13 wherein:
a) the temperature is cooled at 80°C or less after the water strip; and
b) the temperature is further cooled at 60°C or less if the modified pitch is to be stored prior to initiation of step g).

15. The process of claim 1 wherein the alkali metal base is selected from the group consisting of sodium hydroxide, potassium hydroxide or combinations of both.

16. The process of claim 1 wherein the mineral acid is selected from the group consisting of sulfuric acid, hydrochloric acid, phosphoric acid or any combination thereof.

17. The process of claim 1 wherein the solvent in step i) comprises a low molecular weight monohydric alcohol selected from the group consisting of methanol, ethanol and isopropanol.

18. The process of claim 17 wherein the solvent in step i) further comprises acetate esters of methanol, ethanol or isopropanol, ketones or C1 to C8 hydrocarbons, or mixtures thereof.

## Patentansprüche

1. Verfahren zur Isolierung und Reinigung von Phytosterolen und Phytostanolen aus Tallölpech mit folgenden Schritten:
(a) Beschicken einer ersten Destillationssäule mit Tallölpech;
(b) Destillieren des Pechs zur Entfernung überschüssiger Harzsäuren und Fettsäuren zur Herstellung des Pechdestillates;
(c) Verseifen des Pechdestillates mit wässriger Lösung eines oder mehrerer Alkalimetalllaugen zur Herstellung eines verseiften Pechs;
(d) Neutralisation des verseiften Pechs mit ausreichend Säure um Erreichen eines pH-Wertes zwischen 5,8 und 6,3 zur Herstellung des neutralisierten Pechs;
(e) Ermöglichen einer Phasentrennung des neutralisierten Pechs über einen Zeitraum von mindestens 12 Stunden oder bis zu einem Wassergehalt des Pechs von weniger als 15 %, zur Herstellung eines abgetrennten Pechs und einer Wasserphase;
(f) Entfernen im Wesentlichen allen verbleibenden Wassers vom abgetrennten Pech zur Herstellung eines modifizierten Pechs;
(g) Destillation des modifizierten Pechs in einer zweiten Destillationssäule zur Entfernung des leicht flüchtigen Kopfproduktes vom modifizierten Pech und zur Herstellung eines Sumpfproduktes enthaltend freie Phytosterole und/oder Phytostanole;
(h) Destillation nur des Sumpfproduktes in einer dritten Destillationssäule zur Herstellung eines leichtflüchtigen Destillates enthaltend Phytosterole und/oder Phytostanole;
(i) Lösen nur des leichtflüchtigen Destillates in einem Lösungsmittel enthaltend mindestens einen Alkohol zur Herstellung einer Lösung von Phytosterolen und/oder Phytostanolen;
(j) Kühlen der Lösung zur Herstellung einer Schlämme mit darin auskristallisierten Phytosterolen und Phytostanolen;
(k) Waschen, Filtern und Trocknen der Schlämme zur Isolierung der kristallisierten Phytosterolen und/oder Phytostanolen aus dem Filtrat.

2. Verfahren nach Anspruch 1, bei dem im Schritt b) das Pech destilliert wird, um einen Säurenwert kleiner als 40 zu erhalten.

3. Verfahren nach Anspruch 1, bei dem im Schritt b) das Pech destilliert wird, um einen Säurenwert kleiner als 30 zu erhalten.

4. Verfahren nach Anspruch 1, bei dem die Destillationsapparatur in den Schritten b) g) und h) ausgewählt ist aus der Gruppe bestehend aus Kurzwegdestillationsapparatur, Wischfilmverdampferapparatur, Dünnschichtverdampferapparatur und Molekulardestillationsapparatur.

5. Verfahren nach Anspruch 1, bei dem die Destillationsapparatur in den Schritten b) g) und h) eine Wischfilmverdampferapparatur ist.

6. Verfahren nach Anspruch 1, bei dem ein zusätzlicher, paralleler Zustrom in die erste Destillationssäule geführt wird, der aus dem Filtrat des Schrittes k) besteht, **dadurch** charakterisiert dass das Filtrat vorbehandelt ist durch Entfernung der Lösungsmittel und durch Umwandlung im Wesentlichen aller freien Sterole zu Sterolestern.

7. Verfahren nach Anspruch 1, bei dem im Schritt d) das verseifte Pech bei einer Temperatur über 100°C für einen Zeitraum von 1 bis 10 Stunden neutralisiert wird.

8. Verfahren nach Anspruch 1, bei dem die Schritte c) und d) im selben Reaktionsgefäß ablaufen.

9. Verfahren nach Anspruch 1, bei dem Schritt d) unter heftigem Rühren durchgeführt wird.

10. Verfahren nach Anspruch 1, bei dem im Schritt d) Säure und Pech gemischt werden.

11. Verfahren nach Anspruch 1, bei dem Schritt e) ohne Rühren durchgeführt wird.

12. Verfahren nach Anspruch 1, bei dem ein zusätzlicher Schritt nach Schritt e) eingeführt wird, in dem die Wasserphase einer zweiten Phasentrennung unterworfen wird.

13. Verfahren nach Anspruch 1, **dadurch** charakterisiert, dass der Schritt zur Entfernung des übrigen Wassers vom abgetrennten Pech in Schritt f) zur Herstellung des modifizierten Pechs keine Wasserabscheidung beinhaltet, bei der der Druck nicht größer als Athmosphärendruck ist und die Temperatur weniger als 105°C beträgt.

14. Verfahren nach Anspruch 13, bei dem:
a) die Temperatur nach der Wasserabscheidung auf 80°C und weniger heruntergekühlten ist und
b) die Temperatur weiter auf 60°C oder weniger gesenkt wird, wenn das modifizierte Pech vor dem Beginn des Schrittes a) gelagert wird.

15. Verfahren nach Anspruch 1, bei dem die Alkalimetalllauge gewählt ist aus der Gruppe, die gebildet wird von: Natriumhydroxid, Kaliumhydroxid oder der Kombination von beiden.

16. Verfahren nach Anspruch 1, bei dem die Mineralsäure ausgewählt ist aus der Gruppe, die gebildet wird von: Schwefelsäure, Salzsäure, Phosphorsäure oder deren Kombination.

17. Verfahren nach Anspruch 1, bei dem das Lösungsmittel im Schritt i) einen niedrigmolekularen Alkohol enthält, ausgewählt aus der Gruppe, die gebildet wird von Methanol, Ethanol und Isopropanol.

18. Verfahren nach Anspruch 17, bei dem das Lösungsmittel im Schritt i) weiterhin Essigsäureester von Methanol, Ethanol oder Isopropanol, Ketonen oder C1 bis C8 Kohlenwasserstoff oder Mischungen davon enthält.

## Revendications

1. Procédé d'isolement et de purification de phytostérols et de phytostanols à partir de brai de tallol, ledit procédé comprenant :
a) l'introduction du brai dans une première colonne de distillation ;
b) la distillation du brai pour éliminer les acides de colophane et les acides gras en excès et obtenir un brai distillé ;
c) la saponification du brai distillé par une solution aqueuse d'une ou de plusieurs bases métalliques alcalines pour obtenir un brai saponifié ;
d) la neutralisation du brai saponifié par une quantité d'acide suffisante pour obtenir un pH final compris entre 5,8 et 6,3, formant ainsi un brai neutralisé ;
e) la décantation du brai neutralisé pendant une durée d'au moins 12 heures ou jusqu'à ce que la teneur en eau du brai soit inférieure à 15 % après décantation, pour obtenir un brai décanté et une phase aqueuse ;
f) l'élimination de la majorité de l'eau restante du brai décanté pour former un brai modifié ;
g) la distillation du brai modifié dans une seconde colonne de distillation pour éliminer les fractions légères du brai modifié et obtenir une fraction basse comprenant des phytostérols et/ou des phytostanols libres ;
h) la distillation de la fraction basse seule dans une troisième colonne de distillation pour obtenir un distillat de phase légère comprenant des phytostérols et/ou des phytostanols libres ;
i) la dissolution du distillat de phase légère seul dans un solvant comprenant au moins un alcool pour obtenir une solution de phytostérols et/ou de phytostanols ;
j) le refroidissement de la solution pour former une pulpe contenant des phytostérols et/ou des phytostanols cristallisés ; et
k) le lavage, la filtration et le séchage de la pulpe pour isoler les phytostérols et/ou phytostanols cristallisés du filtrat.

2. Procédé selon la revendication 1, dans lequel, dans l'étape b), le brai est distillé pour obtenir un indice d'acidité inférieur à 40.

3. Procédé selon la revendication 1, dans laquel, dans l'étape b), le brai est distillé pour obtenir un indice d'acidité inférieur à 30.

4. Procédé selon la revendication 1, dans lequel les colonnes de distillation des étapes b), g) et h) sont choisies au sein du groupe constitué par les colonnes de distillation de type court, les colonnes d'évaporation à film raclé, les colonnes d'évaporation à couche mince et les colonnes de distillation moléculaire.

5. Procédé selon la revendication 1, dans lequel la colonne de distillation des étapes b), g) et h) est une colonne d'évaporation à film raclé.

6. Procédé selon la revendication 1, dans lequel un courant d'alimentation supplémentaire opposé vient alimenter la première colonne de distillation, ledit courant d'alimentation étant constitué du filtrat de l'étape k), **caractérisé en ce que** le filtrat est prétraité pour en éliminer les solvants et convertir la majorité des stérols libres qu'il contient en esters stéryliques.

7. Procédé selon la revendication 1, dans lequel, dans l'étape d), le brai saponifié est neutralisé à une température supérieure à 100 °C pendant une durée comprise entre 1 et 10 heures.

8. Procédé selon la revendication 1, dans lequel les étapes c) et d) sont effectuées dans le même réacteur.

9. Procédé selon la revendication 1, dans lequel l'étape d) est mise en oeuvre sous agitation vigoureuse.

10. Procédé selon la revendication 1, dans lequel, dans l'étape d), l'acide et le brai sont mélangés.

11. Procédé selon la revendication 1, dans lequel l'étape e) est mise en oeuvre sans agitation.

12. Procédé selon la revendication 1, dans lequel une étape supplémentaire est pratiquée après l'étape e), ladite étape supplémentaire consistant en une seconde décantation de la phase aqueuse.

13. Procédé selon la revendication 1, **caractérisé en ce que** l'étape d'élimination de la majorité de l'eau restante dans le brai décanté de l'étape f) pour obtenir un brai modifié implique l'utilisation d'un processus d'élimination de l'eau dont la pression est inférieure ou égale à la pression atmosphérique et dont la température est inférieure à 105 °C.

14. Procédé selon la revendication 13, dans lequel :
a) la température est laissée à diminuer jusqu'à 80°C ou moins après l'élimination de l'eau ; et
b) la température est à nouveau laissée à diminuer jusqu'à 60°C ou moins si le brai modifié doit être stocké avant le début de l'étape g).

15. Procédé suivant la revendication 1, dans lequel la base métallique alcaline est choisie au sein du groupe constitué par l'hydroxyde de sodium, l'hydroxyde de potassium ou leurs combinaisons.

16. Procédé selon la revendication 1, dans lequel l'acide minéral est choisi au sein du groupe constitué par l'acide sulfurique, l'acide chlorhydrique, l'acide phosphorique ou leurs combinaisons quelconques.

17. Procédé suivant la revendication 1, dans lequel le solvant de l'étape i) comprend un mono-alcool de faible masse moléculaire choisi au sein du groupe constitué par le méthanol, l'éthanol et l'isopropanol.

18. Procédé selon la revendication 17, dans lequel le solvant de l'étape i) comprend en outre des esters acétates de méthanol, d'éthanol ou d'isopropanol, des cétones ou des hydrocarbures en C1-C8, ou leurs mélanges.
